# EUROPÄISCHE PATENTSCHRIFT

(11) **EP 0 700 294 B1**
(45) Veröffentlichungstag und Bekanntmachung des Hinweises auf die Patenterteilung: **05.08.1998**
(21) Anmeldenummer: 94918790.0
(22) Anmeldetag: 25.05.1994
(51) Int. Cl.: A61K 31/65, A61K 47/18

(54) **INJEKTIONSLÖSUNG FÜR DIE INTRAMUSKULÄRE UND SUBKUTANE VERABREICHUNG AN TIERE**
INTRAMUSCULAR AND SUBCUTANEOUS INJECTION SOLUTION FOR ANIMALS
SOLUTION D'ADMINISTRATION DE SUBSTANCES A DES ANIMAUX PAR INJECTION INTRAMUSCULAIRE OU SOUS-CUTANEE

(30) Priorität: 26.05.1993 EP 93108460
(43) Veröffentlichungstag der Anmeldung: 13.03.1996
(73) Patentinhaber: Dörnhöfer, Winfried, D-82269 Geltendorf (DE); Embrechts, Erwin, B-2322 Hoogstraten (BE)
(72) Erfinder: Dörnhöfer, Winfried, D-82269 Geltendorf (DE); Embrechts, Erwin, B-2322 Hoogstraten (BE)
(74) Vertreter: Thiel, Christian, Dr. Dipl.-Chem.
(86) Internationale Anmeldenummer: EP9401696
(87) Internationale Veröffentlichungsnummer: WO9427611

(56) Entgegenhaltungen:
- US-A- 3 128 227
- CHEMICAL ABSTRACTS, vol. 114, no. 9, 4. März 1991, Columbus, Ohio, US; abstract no. 74649, MORAVEK, JOSEF, ET AL. 'DEOXYMYKOIN INJECTION-PHARMACOKINETICS AFTER INTRAVENOUS INFUSION'

## Beschreibung

Die Erfindung betrifft eine Injektionslösung für die subkutane oder intramuskuläre Verabreichung an Tiere mit einem Gehalt, bezogen auf 100 ml Lösung, von 5 bis 30 g Oxytetracylin als Magnesiumkomplex, 0 bis 25 g Polyvinylpyrrolidon, der notwendigen Menge einer organischen Base zur Einstellung eines pH-Wertes von 5,5 bis 9,5 sowie üblichen Zusatzstoffen in einer physiologisch verträglichen wäßrig-organischen Lösemittelphase. Die Erfindung betrifft ferner ein Verfahren zur Herstellung einer solchen Injektionslösung.

In vielen Ländern sind Tetracyclin-Antibiotika Mittel erster Wahl bei zahlreichen therapeutischen Indikationen im veterinären Bereich. Dabei ist für parenterale Anwendungen Oxytetracyclin (OTC) das am meisten verwandte Tetracyclin.

Nichtsdestoweniger ist mit dem Einsatz von Tetracyclinen eine Reihe von ernsthaften Problemen verbunden. So ist der parenterale Einsatz durch ihre schlechte Löslichkeit und geringe Stabilität in wäßrigen Medien beschränkt. Zudem führen sie bei intramuskulärer oder subkutaner Injektion zu starken Reizungen an der Injektionsstelle.

Das Problem der geringen Löslichkeit von Tetracyclinen in Wasser wurde vielfach durch Einsatz von mit Wasser mischbaren Lösungsmitteln und Co-Lösungsmitteln zu lösen versucht. Beispielsweise sind zu nennen Polyethylenglykol, 1,2-Propandiol und N,N-Dimethylacetamid, die ihrerseits stark reizend sind und hämolytisch wirken, NOUWS, Vet. Quart., 1984, 6, 2, 80-84; NOUWS, Vet. Quart., 1990, 12, 3, 129-138; RASMUSSEN et al., Res. vet. Sci., 1976, 20, 55-60; HAPKE et al., DTW, 1983, 90, 161-200 und 216-218.

Bessere galenische Lösungen werden durch die Verwendung von wassermischbaren und besser verträglichen Pyrrolidonderivaten erzielt, die als Lösungsvermittler, Lösungsmittel oder Co-Lösungsmittel in wäßrigen parenteralen Tetracyclinzubereitungen eingesetzt werden. Hier sind Polyvinylpyrrolidon, 2-Pyrrolidon, N-Methylpyrrolidon und 2-Hydroxyethylpyrrolidon zu nennen. Diese Derivate erlauben die Herstellung von Oxytetracyclinformulierungen hoher Konzentration.

Der Vorteil hochkonzentrierter Oxytetracyclinformulierungen mit 20 % OTC liegt darin, daß mit einer einzigen intramuskulären Injektion einer Dosis von 20 mg pro kg Körpergewicht ein antimikrobiell wirksamer Serumspiegel erzeugt werden kann, der bis zu 72 Stunden nach der Injektion anhält - sogenannte Long-acting-Formulierungen (Oxytetracyclin LA). LA-Formulierungen sind aus Praktischen Gründen in der veterinärmedizinischen Anwendung sehr beliebt.

Für die parenterale, perorale und lokale Verabreichung geeignete Lösungen von Oxytetracyclinmagnesiumkomplexen in bis zu 25%igem wäßrigem Polyvinylpyrrolidon mit einem Oxytetracyclingehalt von bis zu 15 % sind aus der GB-A-1 131 007 bekannt. Die dort beschriebenen Lösungen haben einen pH-Wert im Bereich von 8,0 bis 9,5. Der pH-Wert wird mit Hilfe von Natriumhydroxid, Ammoniak, Ethanolamin oder Ethylendiamin eingestellt. Derartige Lösungen sind relativ gut verträglich, haben aber den Nachteil, daß für die parenterale Verabreichung geeignete Formulierungen mit einer höheren OTC-Konzentration als 10 % nicht einstellbar sind. Der LA-Effekt anderer 20 %iger Formulierungen ist hiermit nicht zu erzielen.

Aus der DE-A-26 15 140 ist eine Wirkstofformulierung für die topische transdermale Verabreichung mit einem Trägersystem bekannt, das aus N-Methylpyrrolidon und 2-Pyrrolidon in einem Verhältnis von 1:4 bis 4:1 besteht. Das Trägersystem in der dort beschriebenen Zusammensetzung soll den Durchtritt des auf die Haut aufgebrachten Wirkstoffs durch die Haut fördern. Eine Verabreichung derartiger Formulierungen durch Injektion ist nicht vorgesehen.

Die DE-A-26 59 152 beschreibt für die Injektion geeignete Formulierungen eines Tetracyclinkomplexes in wäßrigem 2-Pyrrolidon, das zugleich Polyvinylpyrrolidon enthalten kann. Die dort beschriebenen Lösungen haben sich jedoch als ausgesprochen gewebereizend erwiesen. Die Injektionen sind für die Tiere häufig schmerzhaft und führen zu unerwünschten Nekrosen. Die Gewebereizungen führen zu hohen und lang andauernden Rückstandswerten im Gewebe und haben unerwünscht lange Wartezeiten zur Folge (Nouws; Rasmussen et al.).

EP-A-0 096 942 beschreibt eine Oxytetracylinzusammensetzung, bei der 10 bis 30 Gew.-Teile Oxytetracyclin in Form eines Erdalkalikomplexes und 2,5 bis 10 Gew.-Teile Polyvinylpyrrolidon zusammen mit Antioxidantien und Basen bei einem pH-Wert von 6,0 bis 9,5 in wäßrigem N-Methylpyrrolidon gelöst sind. Die dort beschriebenen Lösungen sind für die intramuskuläre Injektion bestimmt, zeigen aber die gleiche schlechte örtliche Verträglichkeit, wie die oben beschriebenen 2-Pyrrolidon-Formulierungen (Nouws; Rasmussen et al.).

EP-B-0 271 374 beschreibt ferner die Verwendung von N-Hydroxyethyl-2-pyrrolidon als Lösungsvermittler für Tetracycline und insbesondere auch für Oxytetracyclin. Verträglichkeitsuntersuchungen liegen bislang nicht vor.

Eine weitere OTC-Formulierung in wäßrig-organischen Lösungsmitteln wurde unter der Bezeichnung Tridox vertrieben. Als organische Lösungsmittelkomponente dient eine Mischung aus etwa gleichen Teilen N-Methylpyrrolidon und 2-Pyrrolidon. Die Mischung enthielt Polyvinylpyrrolidon zur Erhöhung der Löslichkeit und zur Verbesserung der Verträglichkeit. Auch diese Formulierung, die für die intramuskuläre Verabreichung bestimmt war, führte an der Injektionsstelle zu Reizungen. Die Resorption und die Serumspiegel waren, auch aufgrund der Reizzustände, nicht optimal.

In allen diesen Formulierungen wird das jeweilige Tetracyclin zunächst an ein Erdalkalimetallion komplexiert, wonach die Formulierung auf einen zuträglichen pH-Wert eingestellt wird. Beides dient der Verbesserung der chemischen und physikalischen Stabilität der Tetracyclin-lösung. Dabei ist zu beachten, daß der optimale pH-Wert für die verschiedenen Tetracyclin-Derivate sehr unterschiedlich sein kann und beispielsweise für Oxytetracyclin zwischen 7,5 und 9,5 liegt.

Weitere Formulierungen, die in den wesentlichen Punkten den oben aufgezeigten entsprechen, sind in der Vergangenheit bekanntgeworden und spiegeln die langjährige Forschungstätigkeit auf diesem Gebiet wieder.

Häufigste Nebenwirkung sind örtliche Reizungen an der Injektionsstelle, die zu Nekrosen und Abkapselungen führen und eine Verminderung oder Verzögerung der Resorption des Wirkstoffes bewirken können. Als Folge ergeben sich auch lange Wartezeiten bis zum Schlachtzeitpunkt und teilweise ein Verwertungsverbot für das um die Injektionsstelle liegende Gewebe.

Amtstierärzten und Inspektoren von Schlachthäusern ist dieses Problem sehr vertraut. Es wurde gefolgert, daß Zubereitungen, die zu starken Nekrosen im Muskelgewebe führen, den heutigen Anforderungen an die veterinärmedizinische Praxis nicht mehr genügen (Vet. Quarterly, 1984, 6, 2, 80 - 84; ibid., 1990, 12, 3, 129 - 138). Nach diesen Veröffentlichungen ist nur eine einzige injizierbare Oxytetracyclinzubereitung hinreichend reizarm eingestellt, nämlich die gemäß GB-A-1 131 007. Bei diesen Zubereitungen sind die Muskelschäden an der Injektionsstelle gering und nach einigen Wochen vollständig verschwunden. In der Praxis hat sich aber gezeigt, daß diese Lösungen aufgrund ihrer hohen Viskosität nur bis zu einem Oxytetracyclingehalt von bis zu 10% injizierbar sind.

Von den Erfindern wurde festgestellt, daß mit den bekannten Präparaten auch sehr wechselnde pharmakokinetische Ergebnisse erzielt werden, die weitgehend mit der jeweiligen örtlichen Reizung korrelieren. So werden bei der intramuskulären Anwendung dieser Formulierungen Serumspiegel erreicht, die manchmal schnell abfallen, manchmal länger anhalten, in jedem Fall aber mit den mehr oder weniger starken Irritationen an den Injektionsstellen zusammenhängen. Dieselben schwankenden Resultate sind auch in der veterinärmedizinischen Literatur beschrieben worden, siehe MEVIUS et al., Vet. quart., 1986, 8, 4, 285-294; NOUWS, Proc. 2nd Congr. Eur. Assoc. vet. Pharmacol. & Toxicol., Toulouse, France, 1982, 195-198; XIA et al., J. vet. Pharmacol. Ther., 1983, 6, 113-120.

Trotz intensiver Forschungstätigkeit ist es aber bislang nicht gelungen, die örtlichen Nebenwirkungen von LA-OTC-Präparat vollständig in den Griff zu bekommen. Reizungsfreie langwirkende OTC-Präparate sind aber in der tierärztlichen Praxis sehr erwünscht, nicht nur aus Gründen der "good veterinary practice" dem zu behandelnden Tier gegenüber, sondern auch weil nur sie einen sicheren und zuverlässigen Serumkurvenverlauf garantieren und Verluste der Injektionsstellen beim Schlachttier vermeiden lassen.

Die bekannten LA-Oxytetracyclin-Formulierungen enthalten das Oxytetracyclin komplexiert an Magnesium, wie bereits erwähnt. Häufig werden zur Herstellung Oxytetracyclin-Hydrochlorid und Magnesiumchlorid verwandt. Der End-pH-Wert der Formulierungen wird mit organischen Basen eingestellt, häufig mit Mono- oder Diethanolamin. In zahlreichen Fällen wird zur Feinabstimmung des pH-Wertes auch Salzsäure zugesetzt. Niedrige Polyvinylpyrrolidonkonzentrationen dienen der Verbesserung der örtlichen Verträglichkeit, wobei aber die Menge durch die gleichzeitige Zunahme der Viskosität begrenzt ist.

Es hat sich jetzt herausgestellt, daß die lokale Reizung an der Injektionsstelle eine Reihe von Ursachen hat. Die Gegenwart von Magnesiumchlorid ist dabei ein wichtiger Faktor. In der Formulierung vorhandenes Magnesiumchlorid kann dabei auf eine unvollständige Komplexierung von Magnesiumionen oder auf die Zugabe von Salzsäure zur Einstellung des pH-Wertes zurückzuführen sein.

Angesichts dessen ist Aufgabe der Erfindung die Bereitstellung von OTC-Injektionslösungen mit verminderter Tendenz zur Erzeugung von lokalen Reizzuständen bei intramuskulärer oder subkutaner Injektion. Zugleich sollen diese Lösungen gegenüber den bisher bekannten Präparaten wenigstens gleichwertige Serumspiegel und eine möglichst vollständige Ausscheidung aus dem Körper gewährleisten.

Diese Aufgabe wird mit einer Injektionslösung der eingangs genannten Art gelöst, die im wesentlichen chloridionenfrei eingestellt ist und als organische Base zur Einstellung des pH-Wertes eine basische Aminosäure enthält.

Erfindungsgemäß wird der Einsatz von Chloridionen bei der Formulierung vermieden, d.h. weder das Oxytetracyclin noch die zur Komplexierung verwandte Magnesiumverbindung werden als Chlorid zugesetzt. Ebensowenig wird Salzsäure zur Korrektur des pH-Wertes verwandt. Das Oxytetracyclin wird zweckmäßigerweise als Base zugesetzt, insbesondere als Dihydrat. Als Magnesiumverbindung wird vorzugsweise Magnesiumoxid MgO verwandt.

Zur weiteren Verbesserung der lokalen Verträglichkeit der erfindungsgemäßen Injektionslösungen kann es vorteilhaft sein, das zur Komplexierung des OTC verwandte Magnesium unterstoichiometrisch einzusetzen, etwa in einem Verhältnis von 0,75:1 bis < 1:1, vorzugsweise in einer Menge von 0,80:1 bis 0,95:1, jeweils gegenüber Oxytetracyclin. Höhere Konzentrationen an freien Magnesiumverbindungen und insbesondere auch MgO sind ebenfalls gewebereizend.

Es ist bekannt, daß Polyvinylpyrrolidon die lokale Verträglichkeit von Tetracyclinen verbessern kann. Es ist deshalb erwähnenswert, daß die erfindungsgemäßen Injektionslösungen auch ohne Zusatz von Polyvinylpyrrolidon eine bessere lokale Verträglichkeit zeigen als bekannte Präparate, die Polyvinylpyrrolidon enthalten. Ein Verzicht auf den Zusatz von Polyvinylpyrrolidon kann insbesondere deshalb von Vorteil sein, weil mit solchem Zusatz die Viskosität der Injektionslösung zunimmt. Eine zu hohe Viskosität erschwert die Spritzbarkeit der Formulierung.

Wird Polyvinylpyrrolidon zugesetzt, hat dieses zweckmäßigerweise ein Molekulargewicht im Bereich von 5.000 bis 30.000. Besonders bevorzugt sind Molekulargewichte von 10.000 bis 17.000. Geeignet sind insbesondere solche mit K-Werten von 12 bis 30, beispielsweise K12 bis K17.

In erfindungsgemäßen Injektionslösungen ist Polyvinylpyrrolidon in einer Menge von 0 bis 25 g/100 ml enthalten, vorzugsweise in einer Menge von 2,5 bis 20 g/100 ml Lösung. Am besten eignet sich für die Injektionslösung ein Polyvinylpyrrolidon mit niedrigem Molekulargewicht, beispielsweise das Kollidon 12 PF der Fa. BASF mit einem K-Wert von 12, in einer Konzentration von etwa 2,5 bis 10 g/100 ml.

Zur Erhöhung der Verträglichkeit der erfindungsgemäßen Injektionslösungen hat es sich als vorteilhaft erwiesen, das Polyvinylpyrrolidon in Wasser unter Druck bei einer Temperatur von mehr als 115 °C in Lösung zu bringen. Der Zeitraum dieses Autoklavierungsprozesses soll wenigstens etwa 15 min betragen, zweckmäßigerweise wenigstens etwa 30 min. Zur Stabilisierung und Vermeidung von Verfärbungen kann der Lösung während des Autoklavierungsprozesses Natriummetabisulfit, etwa in einer Menge bis zu 0,5 Gew.% und vorzugsweise von etwa 0,1 Gew.%, bezogen auf das Polyvinylpyrrolidon, zugesetzt werden. Durch die Autoklavierung der Lösung wird zudem die Spritzbarkeit auch von hochkonzentrierten Lösungen um etwa bis zu 20 % verbessert.

Der Wirkstoff Oxytetracyclin ist in den erfindungsgemäßen Injektionslösungen in einer Menge von 5 bis 30 g, insbesondere aber in einer Menge von etwa 20 g/100 ml Lösung enthalten.

In den bisher patentierten Formulierungen wurden organischen Basen zur Einstellung des pH-Wertes eingesetzt, überwiegend Mono- oder Diethanolamin. Diese Produkte sind sehr reizend, wie aus Studien von T. SADO hervorgeht (Nippon Nogeikagaku Kaishi, 1961, 35, 1164-1177). Es wurde gefunden, daß basische Aminosäuren wie l-Arginin, L-Lysin oder L-Ornithin geeignete Substitutionsprodukte mit ausgezeichneter lokaler Verträglichkeit sind.

Der für die erfindungsgemäße Injektionslösung gewünschte pH-Wert im Bereich von 5,0 bis 9,5 vorzugsweise 6,0 bis 9,5, wird erfindungsgemäß mit einer basischen Aminosäure eingestellt. Es können natürlich vorkommende oder synthetisch erzeugte Aminosäuren verwandt werden, wobei in jedem Fall die physiologisch verträglichere L-Form bevorzugt ist. Erfindungsgemäß wird unter einer basischen Aminosäure eine solche verstanden, die wenigstens 2 Aminofunktionen zusammen mit wenigstens einer Säurefunktion enthält, wobei die Zahl der Aminofunktionen die der Säurefunktionen übersteigt. Als Säurefunktion wird in der Regel eine Carbonsäurefunktion verstanden, jedoch können auch andere Säurefunktionen den gewünschten Zweck erfüllen, beispielsweise eine Sulfonsäurefunktion.

Besonders bevorzugt ist die Verwendung der natürlichen basischen Aminosäuren L-Arginin, L-Lysin und L-Ornithin. Auf diese Weise wird das mit der Verwendung von Natriumhydroxid oder organischen Aminen verbundene Reizpotential vermieden.

Es hat sich gezeigt, daß die Verwendung basischer Aminosäuren die Verträglichkeit der damit hergestellten Formulierungen deutlich verbessert. Es kann aber zweckmäßig sein, zusätzliche konventionelle Basen in geringen Mengen zuzusetzen, um die pH-Einstellung zu erleichtern oder das Ausfallen der Aminosäure wegen Überschreitung der Löslichkeit zu vermeiden.

Die gute Verträglichkeit der erfindungsgemäßen Injektionslösungen wird weiterhin durch die Verwendung chloridionenfreier Ausgangsmaterialien bestimmt. Die hier verwandten Begriffe "chloridionenfrei" bzw. "im wesentlichen chloridionenfrei" bedeuten, daß der Chloridionenanteil sich in einer Größenordnung bewegt, die keine physiologischen Reizzustände mehr hervorrufen kann.

Im Grunde genommen können die erfindungsgemäßen Lösungen aus beliebigen, auch chloridhaltigen Ausgangsstoffen hergestellt werden. Für die chloridionenfreie Einstellung müssen dann Maßnahmen getroffen werden, unzuträgliche Chloridmengen aus der fertigen Lösung zu entfernen. Dies kann beispielsweise dadurch erfolgen, daß man die Magnesiumkomplexe zwar ausgehend von chloridionenhaltigen Ausgangsstoffen, wie Hydrochloriden und Magnesiumchlorid, darstellt, die dabei anfallenden Chloride jedoch abtrennt, beispielsweise durch Kristallisation. Um ein im wesentlichen chloridionenfreies Produkt zu erhalten, ist es jedoch grundsätzlich bevorzugt, ein chloridionenfreies Magnesiumsalz zu verwenden, insbesondere Magnesiumoxid. Oxytetracyclin wird vorzugsweise in Form der freien Base, insbesondere als Dihydrat eingesetzt. Weiterhin können chloridionenfreie Salze verwandt werden, deren Verträglichkeit und Reizarmut bekannt ist.

Die erfindungsgemäßen Injektionslösungen werden vorzugsweise auf eine Spritzviskosität von weniger als 70 cps eingestellt. Besonders bevorzugt ist eine Spritzviskosität von weniger als 40 cps.

Für die wäßrig-organische Lösungsmittelphase können für Oxytetracyclinformulierungen übliche organische Lösungsmittel eingesetzt werden. Voraussetzung ist ihre physiologische Verträglichkeit. Als geeignet haben sich Pyrrolidon und seine Derivate erwiesen, insbesondere N-Methylpyrrolidon, 2-Pyrrolidon und N-(2-Hydroxyethyl)Pyrrolidon. Weiter sind zu nennen N,N-Dimethylacetamid, Hydroxyethylacetamid, und andere übliche Säureamid-Derivate, Glycerolformal, Polyethylenglykol und Polypropylenglykol. Bevorzugte wäßrig-organische Lösungsmittel sind N-Methylpyrrolidon und 2-Pyrrolidon.

Die erfindungsgemäßen Injektionslösungen sind sowohl mit den einzelnen Lösungsmitteln, als auch mit ihren Mischungen herzustellen. Es wurde gefunden, daß insbesondere Mischungen zweier verschiedener Lösungsmittel vom Pyrrolidontyp die Resorptionscharakteristiken gegenüber der Verwendung der jeweiligen Lösungsmittel allein weiter verbessert. Hinweise auf diesen Effekt befinden sich bereits in der DE-A-2 615 140, dort aber für den Fall transdermaler Formulierungen. Der gleiche Effekt tritt aber auch bei subkutaner oder intramuskulärer Verabreichung ein.

Die erfindungsgemäßen Injektionslösungen enthalten zweckmäßigerweise 8 bis 66 g Lösungsmittel auf 100 ml Lösung.

Als besonders geeignet für die intramuskuläre Verabreichung haben sich Formulierungen mit einem Oxytetracyclingehalt von etwa 10 bis 30g, insbesondere etwa 20 g auf 100 ml Lösung erwiesen. Eine bevorzugte Formulierung enthält in der Lösungsmittelphase 20 bis 60 g N-Methylpyrrolidon und 2-Pyrrolidon in einem Mischungsverhältnis von 92:8 bis 70:30. Zur Stabilisierung der Lösung und Erhöhung der Verträglichkeit können 2,5 bis 10 g Polyvinylpyrrolidon auf 100 ml Lösung zugesetzt werden.

Vorzugsweise ist dabei das Gewichtsverhältnis von N-Methylpyrrolidon zu 2-Pyrrolidon kleiner als 90:10 und größer als 80:20, und beträgt insbesondere etwa 5:1 bis 4:1. Es hat sich herausgestellt, was bei Einhaltung dieser Grenzen ein Optimum an Lösungseffekt bei guter Spritzbarkeit und guter Verträglichkeit erzielt wird. Zugleich garantiert die Mischung eine gute Resorption des intramuskulär oder subkutan gespritzten Arzneimittels, so daß frühzeitig die gewünschten hohen Serumspiegel erreicht werden. Geweberückstände werden binnen kurzer Zeit vollständig abgebaut, so daß sich eine Verkürzung der bisher einzuhaltenden Wartezeiten ergibt.

Die ausgezeichneten pharmakokinetischep Eigenschaften der ordnungsgemäßen Injektionslösungen wurden durch Untersuchung klassischer Serumkurven bestätigt. Die Serum-Kreatin-Phosphokinasewerte (CK) ergaben eine ausgezeichnete, den bisher bekannten Präparaten überlegene lokale Verträglichkeit, die vom bekannten "Shintani-Test" bestätigt wurde.

Die pharmakokinetischen Eigenschaften der erfindungsgemäßen Lösungen wurden durch Bestimmung der klassischen Serumkurven an Kälbern untersucht. Die Oxytetracyclin-Serumbestimmung erfolgte dabei mittels Hochdruckflüssigkeitschromatografie.

Es ist bekannt, daß Schädigungen der Skelettmuskulatur eine Erhöhung der CK-Aktivität im Serum verursachen. Die CK-Aktivität steigt je nach Umfang der Traumatisierung, beispielsweise auch nach der intramuskulären Injektion bestimmter Arzneimittel, an (veterinärmedizinische Laboruntersuchungen für die Diagnose und Verlaufskontrolle, Boehringer Mannheim; UCELLI et al., Rec. med. vet., 1988, 164, 11, 939-943).

Der Anstieg der CK-Werte wurde an Kälbern nach einmaliger intramuskulärer Verabreichung verschiedener Präparate untersucht. In jeder Versuchsgruppe wurden nach dem Zufallsprinzip 4 Kälber ausgewählt, wobei Geschlecht (weiblich), Rasse (schwarzbunt) und Körpergewicht (110±2 kg) übereinstimmten. Die Tiere wurden einmalig intramuskulär behandelt, wobei die therapeutische Dosis ein ml Präparat pro 10 kg Körpergewicht (20 mg Oxytetracyclin pro kg Körpergewicht) betrug. Blutproben wurden unmittelbar vor sowie 8, 12, 24, 48, 60, 72 und 96 Stunden nach der Injektion entnommen. Die CK-Bestimmungen erfolgten mittels automatisierter Analyse gemäß Boehringer Mannheim mit einem BM/Hitachi 704 Gerät. Als Formulierung wurde die des erfindungsgemäßen Beispiels 7 mit einem kommerziell verfügbaren Präparat (20 % Oxytetracyclin in wäßrigem 2-Pyrrolidon) verglichen.

| | Stunden post Injektionem | | | | | | |
|---|---|---|---|---|---|---|---|
| CK-Werte in IU/l (Mittelwerte) | 0 | 8 | 12 | 24 | 48 | 72 | 96 |
| Formel Beispiel 7 kommerzielles | 18 | 79 | 55 | 34 | 23 | 22 | 20 |
| Oxytetracyclin 20% in 2-Pyrr. | 22 | 291 | 163 | 98 | 58 | 49 | 26 |

Aus den Ergebnissen ist ersichtlich, daß die Injektionslösung gemäß Beispiel 7 nur eine etwa 4-fache Erhöhung der CK-Werte ergab, die nach 48 Stunden im wesentlichen vollständig abgeklungen war. Im Vergleichspräparat trat dagegen eine etwa 13-fache Erhöhung der CK-Werte auf, die erst nach 92 Stunden wieder abgeklungen war.

Eine Überprüfung nach dem Shintani-Test (Shintani et al., Tox. appl. Pharmacol., 1967, 11, 293-301) bestätigte die Ergebnisse des CK-Tests. Der Reizungsgrad wurde anhand einer makroskopischen Bewertung der Injektionsstelle nach intramuskulärer Verabreichung an Kaninchen bestimmt.

| | Stunden post Injektionem | |
|---|---|---|
| SHINTANI SCORE (Mittelwert von je 3 Tieren) | 48 | 72 |
| Formel Beispiel 3 | 3,00 (*) | 2,00 |
| kommerzielles Oxytetracyclin 20% N-Methylpyrrolidon | 4,33 | 3,33 |
| Die Shintani-Kriterien sind: | | |
| Keine Reaktion: | 0 bis 0,4 | |
| Geringfügige Reaktion: | 0,5 bis 1,4 | |
| Milde Reaktion: | 1,5 bis 2,4 | |
| Mäßige Reaktion: | 2,5 bis 3,4 | |
| Starke Reaktion: | 3,5 bis 4,4 | |
| Ernsthafte Reaktion: | 4,5 bis 5 | |

| | | |
|---|---|---|
| (*) 3,00 nur wegen gelber Präzipitation. Die Injektionsstelle war aber frei von Nekrosen. | | |

Die erfindungsgemäßen Formulierungen werden vorzugsweise nach einem Verfahren hergestellt, bei dem in einem ersten Schritt die erforderliche Menge Polyvinylpyrrolidon in Wasser gelöst und über einen Zeitraum von wenigstens 15 min bei einer Temperatur von mehr als 115°C autoklaviert wird, diese Lösung nach dem Abkühlen in einem zweiten Schritt mit einem oder mehreren organischen Lösungsmitteln, die physiologisch zuträglich sind, Stabilisierungsmitteln und einem chloridfreien Magnesiumsalz, jeweils in den erforderlichen Mengen versetzt und bis zur Homogenität gerührt wird, danach in einem dritten Schritt die erforderliche Menge chloridionenfreies Oxytetracyclin oder Oxytetracyclin-Derivat eingerührt wird, wobei durch Zufügen der basischen Aminosäure der gewünschte pH-Wert eingestellt wird. Für den Fall, daß in der erfindungsgemäßen Formulierung kein Polyvinylpyrrolidon zugegen ist, wird natürlich auf den Autoklavierungsschritt verzichtet.

Die Erfindung wird durch die nachstehenden Beispiele näher erläutert.

### Herstellungsverfahren

Die nachfolgenden Beispiele 1 bis 8 wurden mit dem folgenden Herstellungsverfahren durchgeführt.

Sofern zugegeben, wurde das Polyvinylpyrrolidon zusammen mit dem Natriummetabisulfit in etwa 25 ml Wasser für die Injektion eingemischt und bei 121 °C 30 min lang autoklaviert. Nach dem Abkühlen auf Raumtemperatur wurde die organische Lösungsmittelphase (2-Pyrrolidon und/oder N-Methylpyrrolidon) zugemischt, anschließend das Magnesiumoxid. Es wurde gemischt, bis eine homogene Aufschlämmung erhalten wurde.

Die so erhaltene Mischung wurde nachfolgend mit Natriumformaldehydsulfoxylat und der basischen Aminosäure versetzt. Es wurde auf 50 °C erwärmt und langsam, unter fortsetzendem Rühren, mit Oxytetracyclindihydrat versetzt. Es wurde so lange erwärmt, bis das Oxytetracyclin vollständig komplexiert war.

Nach dem Abkühlen auf Raumtemperatur wurde mit Wasser für Injektionszwecke auf 100 ml verdünnt. Die Lösung wurde steril gefiltert und unter Stickstoff als Schutzgas in Ampullen abgefüllt.

| Beispiel 1 | |
|---|---|
| Oxytetracyclindihydrat (entspr. 20 g Oxytetracyclin) | 21.600 g |
| Magnesiumoxid | 1.600 g |
| Polyvinylpyrrolidon (K17) | 5.000 g |
| Natriummetabisulfit | 0.005 g |
| 2-Pyrrolidon | 8.880 g |
| N-Methylpyrrolidon | 41.200 g |
| Natriumformaldehydsulfoxylat | 0.400 g |
| L-Arginin | 3.250 g |
| Wasser für Injektionszwecke | ad 100 ml |
| Der pH-Wert der Lösung betrug 8.90. | |

| Beispiel 2 | |
|---|---|
| Oxytetracyclindihydrat (entspr. 20 g Oxytetracyclin) | 21.600 g |
| Magnesiumoxid | 1.600 g |
| Polyvinylpyrrolidon (K17) | 5.000 g |
| Natriummetabisulfit | 0.005 g |
| 2-Pyrrolidon | 50.000 g |
| Natriumformaldehydsulfoxylat | 0.400 g |
| L-Arginin | 3.650 g |
| Wasser für Injektionszwecke | ad 100 ml |
| Der pH-Wert der Lösung betrug 8.74. | |

| Beispiel 3 | |
|---|---|
| Oxytetracyclindihydrat (entspr. 20 g Oxytetracyclin) | 21.600 g |
| Magnesiumoxid | 1.600 g |
| Polyvinylpyrrolidon (K17) | 8.000 g |
| Natriummetabisulfit | 0.008 g |
| 2-Pyrrolidon | 8.880 g |
| N-Methylpyrrolidon | 41.200 g |
| Natriumformaldehydsulfoxylat | 0.400 g |
| L-Arginin | 3.250 g |
| Wasser für Injektionszwecke | ad 100 ml |
| Der pH-Wert der Lösung betrug 8.90. Die Viskosität bei 25°C war 45 cst. | |

| Beispiel 4 | |
|---|---|
| Oxytetracyclindihydrat (entspr. 20 g Oxytetracyclin) | 21.600 g |
| Magnesiumoxid | 1.450 g |
| 2-Pyrrolidon | 8.880 g |
| N-Methylpyrrolidon | 41.200 g |
| Natriumformaldehydsulfoxylat | 0.400 g |
| L-Arginin | 3.750 g |
| Wasser für Injektionszwecke | ad 100 ml |
| Der pH-Wert der Lösung betrug 8.73. | |

| Beispiel 5 | |
|---|---|
| Oxytetracyclindihydrat (entspr. 20 g Oxytetracyclin) | 21.600 g |
| Magnesiumoxid | 1.600 g |
| Polyvinylpyrrolidon (K17) | 8.000 g |
| Natriummetabisulfit | 0.008 g |
| 2-Pyrrolidon | 8.880 g |
| N-Methylpyrrolidon | 41.200 g |
| Natriumformaldehydsulfoxylat | 0.400 g |
| L-Cystein | 0.025 g |
| L-Arginin | 3.250 g |
| Wasser für Injektionszwecke | ad 100 ml |
| Der pH-Wert der Lösung betrug 8.77. Die Viskosität bei 25°C war 44 cst. | |

| Beispiel 6 | |
|---|---|
| Oxytetracyclindihydrat (entspr. 20 g Oxytetracyclin) | 21.600 g |
| Magnesiumoxid | 1.450 g |
| Polyvinylpyrrolidon (K12) | 8.000 g |
| Natriummetabisulfit | 0.008 g |
| 2-Pyrrolidon | 8.880 g |
| N-Methylpyrrolidon | 41.200 g |
| Natriumformaldehydsulfoxylat | 0.400 g |
| L-Arginin | 3.750 g |
| Wasser für Injektionszwecke | ad 100 ml |
| Der pH-Wert der Lösung betrug 8.77. | |

| Beispiel 7 | |
|---|---|
| Oxytetracyclindihydrat (entspr. 20 g Oxytetracyclin) | 21.600 g |
| Magnesiumoxid | 1.450 g |
| Polyvinylpyrrolidon (K17) | 2.500 g |
| Natriummetabisulfit | 0.002 g |
| 2-Pyrrolidon | 8.880 g |
| N-Methylpyrrolidon | 41.200 g |
| Natriumformaldehydsulfoxylat | 0.400 g |
| L-Arginin | 3.750 g |
| Wasser für Injektionszwecke | ad 100 ml |
| Der pH-Wert der Lösung betrug 8.83. | |

| Beispiel 8 | |
|---|---|
| Oxytetracyclindihydrat (entspr. 30 g Oxytetracyclin) | 33.570 g |
| Magnesiumoxid | 2.300 g |
| Polyvinylpyrrolidon (K17) | 8.000 g |
| Natriummetabisulfit | 0.008 g |
| 2-Pyrrolidon | 9.000 g |
| N-Methylpyrrolidon | 49.000 g |
| Natriumformaldehydsulfoxylat | 0.400 g |
| L-Arginin | 6.500 g |
| Wasser für Injektionszwecke | ad 100 ml |
| Der pH-Wert der Lösung betrug 8.60. | |

## Patentansprüche

1. Injektionslösung für die intramuskuläre oder subkutane Verabreichung an Tiere mit einem Gehalt, bezogen auf 100 ml Lösung, von 5 bis 30 g Oxytetracyclin als Magnesiumkomplex, 0 bis 25 g Polyvinylpyrrolidon, der notwendigen Menge einer organischen Base zur Einstellung eines pH-Wertes von 5,5 bis 9,5, sowie üblichen Zusatzstoffen in einer wäßrig-organischen Lösemittelphase, dadurch gekennzeichnet, daß die Injektionslösung im wesentlichen chloridionenfrei eingestellt ist und als organische Base zur Einstellung des pH-Wertes eine basische Aminosäure enthält.

2. Injektionslösung nach Anspruch 1, dadurch gekennzeichnet, daß sie 2,5 bis 20 g, bezogen auf 100 ml Lösung, Polyvinylpyrrolidon mit einem Molekulargewicht von 5.000 bis 30.000, vorzugsweise von 10.000 bis 17.000, enthält.

3. Injektionslösung nach Anspruch 2, dadurch gekennzeichnet, daß das Polyvinylpyrrolidon einen K-Wert von 12 bis 30 hat.

4. Injektionslösung nach Anspruch 2 oder 3, hergestellt unter Verwendung einer Polyvinylpyrrolidonlösung in Wasser, die durch Autoklavieren vorzugsweise bei über 115°C für wenigstens 15 min erhalten wurde.

5. Injektionslösung nach Anspruch 4, dadurch gekennzeichnet, daß die Polyvinylpyrrolidonlösung durch Autoklavieren in Gegenwart von Natriummetabisulfit in einer Menge von bis zu 0,5 Gew.-%, vorzugsweise von etwa 0,1 Gew.-%, bezogen auf das Polyvinylpyrrolidon, erhalten wurde.

6. Injektionslösung nach einem der vorstehenden Ansprüche, dadurch gekennzeichnet, daß sie als basische Aminosäure L-Arginin, L-Lysin, L-Ornithin oder Mischungen davon enthält.

7. Injektionslösung nach einem der vorstehenden Ansprüche mit einer Spritzviskosität von weniger als 70 cps, vorzugsweise weniger als 40 cps.

8. Injektionslösung nach einem der Ansprüche 1 bis 7 für die intramuskuläre Verabreichung mit einem Oxytetracyclingehalt von etwa 20 g auf 100 ml Lösung.

9. Injektionslösung nach einem der vorstehenden Ansprüche, dadurch gekennzeichnet, daß sie als organische Lösungsmittelphase 2-Pyrrolidon, N-Methylpyrrolidon, N-Hydroxyethylpyrrolidon oder Mischungen davon enthält.

10. Injektionslösung nach Anspruch 9, gekennzeichnet durch einen Gehalt von 8 bis 66 g N-Methylpyrrolidon und 2-Pyrrolidon in einem Gewichtsverhältnis von 92:8 bis 70:30, insbesondere 90:10 bis 80:20, auf 100 ml Lösung.

11. Injektionslösung nach einem der Ansprüche 1 bis 10, gekennzeichnet durch ein molares Verhältnis von Magnesium zu Oxytetracyclin von 0,80:1 bis 0,95:1.

12. Verfahren zur Herstellung einer Injektionslösung nach einem der vorstehenden Ansprüche, dadurch gekennzeichnet, daß in einem ersten Schritt die erforderliche Menge Polyvinylpyrrolidon in Wasser gelöst und über einen Zeitraum von wenigstens 15 min bei einer Temperatur von mehr als 115°C autoklaviert wird, diese Lösung nach dem Abkühlen in einem zweiten Schritt mit einem oder mehreren physiologisch verträglichen wassermischbaren organischen Lösungsmitteln, Stabilisierungsmitteln und einer chloridfreien Magnesiumverbindung, jeweils in den erforderlichen Mengen, versetzt und bis zur Homogenität gerührt wird und danach in einem dritten Schritt die erforderliche Menge chloridionenfreies Oxytetracyclin oder Oxytetracyclinderivat eingerührt wird, der gewünschte pH-Wert durch Zugabe einer basischen Aminosäure eingestellt wird.

13. Verfahren nach Anspruch 12, dadurch gekennzeichnet, daß als Ausgangsmaterial Oxytetracyclin als Base, Magnesiumoxid und L-Arginin verwandt werden.

14. Verfahren nach Anspruch 13, dadurch gekennzeichnet, daß das Magnesiumoxid unterstoichiometrisch verwandt wird, vorzugsweise in einem Verhältnis von 0,80:1 bis 0,95:1 zum Oxytetracyclin.

## Claims

1. Injection solution for intramuscular or subcutaneous administration to animals with a content, based on 100 ml of solution, of 5 to 30 g of oxytetracycline as magnesium complex, 0 to 25 g of polyvinylpyrrolidone, the required amount of an organic base to adjust a pH of 5.5 to 9.5, and conventional additives in an aqueous/organic solvent phase, characterized in that the injection solution is adjusted to be essentially free of chloride ions and contains a basic amino acid as organic base to adjust the pH.

2. Injection solution according to Claim 1, characterized in that it contains 2.5 to 20 g, based on 100 ml of solution, of polyvinylpyrrolidone with a molecular weight of 5000 to 30,000, preferably of 10,000 to 17,000.

3. Injection solution according to Claim 2, characterized in that the polyvinylpyrrolidone has a K value of 12 to 30.

4. Injection solution according to Claim 2 or 3, produced using a polyvinylpyrrolidone solution in water, which has been obtained by autoclaving, preferably at above 115°C, for at least 15 min.

5. Injection solution according to Claim 4, characterized in that the polyvinylpyrrolidone solution has been obtained by autoclaving in the presence of sodium metabisulphite in an amount of up to 0.5% by weight, preferably of about 0.1% by weight, based on the polyvinylpyrrolidone.

6. Injection solution according to any of the preceding claims, characterized in that it contains L-arginine, L-lysine, L-ornithine or mixtures thereof as basic amino acid.

7. Injection solution according to any of the preceding claims with an injection viscosity of less than 70 cps, preferably less than 40 cps.

8. Injection solution according to any of Claims 1 to 7 for intramuscular administration with an oxytetracycline content of about 20 g per 100 ml of solution.

9. Injection solution according to any of the preceding claims, characterized in that it contains 2-pyrrolidone, N-methylpyrrolidone, N-hydroxyethylpyrrolidone or mixtures thereof as organic solvent phase.

10. Injection solution according to Claim 9, characterized by a content of 8 to 66 g of N-methylpyrrolidone and 2-pyrrolidone in a weight ratio of 92:8 to 70:30, in particular 90:10 to 80:20, per 100 ml of solution.

11. Injection solution according to any of Claims 1 to 10, characterized by a molar ratio of magnesium to oxytetracycline of 0.80:1 to 0.95:1.

12. Process for the production of an injection solution according to any of the preceding claims, characterized in that, in a first step, the required amount of polyvinylpyrrolidone is dissolved in water and autoclaved at a temperature of more than 115°C for a period of at least 15 min, in a second step this solution is, after cooling, mixed with one or more water-miscible organic solvents which are physiologically advantageous, stabilizers and a chloride-free magnesium compound in each case in the required amounts, and stirred to homogeneity, and then, in a third step, the required amount of oxytetracycline or oxytetracycline derivative, which is free of chloride ions, is stirred in, with the required pH being adjusted by adding a basic amino acid.

13. Process according to Claim 12, characterized in that oxytetracycline as base, magnesium oxide and L-arginine are used as starting material.

14. Process according to Claim 13, characterized in that less than the stoichiometric amount of magnesium oxide is used, preferably in a ratio of 0.80:1 to 0.95:1 to the oxytetracycline.

## Revendications

1. Solution injectable destinée à l'administration intramusculaire ou sous-cutanée chez l'animal, contenant, pour 100 ml de solution, de 5 à 30 g d'oxytétracycline sous forme de complexe avec le magnésium, de 0 à 25 g de polyvinylpyrrolidone, la quantité nécessaire d'une base organique pour ajuster le pH entre 5,5 et 9,5, ainsi que les additifs usuels dans une phase aqueuse de solvant organique, caractérisée en ce que la solution injectable est ajustée de façon à être sensiblement exempte d'ions chlorure et contient un acide aminé basique comme base organique destinée à l'ajustement du pH.

2. Solution injectable selon la revendication 1, caractérisée en ce qu'elle contient 2,5 à 20 g, par rapport à 100 ml de solution, de polyvinylpyrrolidone ayant une masse moléculaire comprise entre 5 000 et 30 000, de préférence entre 10 000 et 17 000.

3. Solution injectable selon la revendication 2, caractérisée en ce que la polyvinylpyrrolidone a une valeur K de 12 à 30.

4. Solution injectable selon la revendication 2 ou 3, préparée par utilisation d'une solution aqueuse de polyvinylpyrrolidone obtenue de préférence par passage à l'autoclave à une température de plus de 115°C pendant au moins 15 s.

5. Solution injectable selon la revendication 4, caractérisée en ce que la solution de polyvinylpyrrolidone a été obtenue par passage à l'autoclave en présence de métabisulfite de sodium en une quantité atteignant jusqu'à 0,5 % en poids, de préférence égale à environ 0,1 % en poids, par rapport à la polyvinylpyrrolidone.

6. Solution injectable selon une des revendications précédentes, caractérisée en ce qu'elle contient comme acide aminé basique de la L-arginine, de la L-lysine, de la L-ornithine ou des mélanges de ces dernières.

7. Solution injectable selon une des revendications précédentes, ayant une viscosité à la pulvérisation inférieure à 70 cP, de préférence inférieure à 40 cP.

8. Solution injectable selon une des revendications 1 à 7, destinée à l'administration intramusculaire, ayant une teneur en oxytétracycline d'environ 20 g pour 100 ml de solution.

9. Solution injectable selon une des revendications précédentes, caractérisée en ce qu'elle contient comme phase de solvant organique de la 2-pyrrolidone, de la N-méthylpyrrolidone, de la N-hydroxyéthylpyrrolidone ou des mélanges des ces dernières.

10. Solution injectable selon la revendication 9, caractérisée par une teneur de 8 à 66 g de N-méthylpyrrolidone et de 2-pyrrolidone dans un rapport pondéral de 92:8 à 70:30, notamment de 90:10 à 80:20, pour 100 ml de solution.

11. Solution injectable selon une des revendications 1 à 10, caractérisée par un rapport molaire du magnésium à l'oxytétracycline de 0,80:1 à 0,95:1.

12. Procédé de production d'une solution injectable selon une des revendications précédentes, caractérisé en ce qu'au cours d'une première étape on dissout dans de l'eau la quantité nécessaire de polyvinylpyrrolidone et qu'on passe à l'autoclave pendant une durée d'au moins 15 min à une température de plus de 115°C, qu'au cours d'une deuxième étape on ajoute à cette solution après refroidissement un ou plusieurs solvants organiques miscibles à l'eau, physiologiquement tolérés, des stabilisants et un composé de magnésium exempt de chlorure, en les quantités respectivement nécessaires, et qu'on agite jusqu'à homogénéitè et qu'ensuite, au cours d'une troisième étape, on délaie la quantité nécessaire d'oxytétracycline ou de dérivés d'oxytétracycline exempts d'ions chlorure et qu'on ajuste au pH souhaité par addition d'un acide aminé basique.

13. Procédé selon la revendication 12, caractérisé en ce qu'on utilise comme substances de départ de l'oxytétracycline sous forme de base, de l'oxyde de magnésium et de la L-arginine.

14. Procédé selon la revendication 13, caractérisé en ce qu'on utilise l'oxyde de magnésium en une quantité inférieure à la quantité stoechiométrique, de préférence dans un rapport de 0.80:1 à 0,95:1 par rapport à l'oxytétracycline.
